# EUROPEAN PATENT APPLICATION

(11) **EP 2 491 930 A1**
(43) Date of publication of application: **29.08.2012**
(21) Application number: 11155916.7
(22) Date of filing: 25.02.2011
(51) Int. Cl.: A61K 31/4402, A61K 31/495, A61P 27/16, A61K 9/00, A61K 9/14, A61K 9/20, A61K 9/48, A61K 9/50

(54) **Pharmaceutical combination of betahistine and trimetazidine**

(71) Applicant: Deva Holding Anonim Sirketi, 34303 Istanbul (TR)
(72) Inventor: Haas, Philipp Daniel, 34303, ISTANBUL (TR); Koc, Fikret, 34303, ISTANBUL (TR); Sivasligil, Ramazan, 34303, ISTANBUL (TR); Kandemir, Levent, 34303, ISTANBUL (TR)

(57) **Abstract**

This invention is related to pharmaceutical combination of betahistine or pharmaceutically acceptable salts thereof and trimetazidine or pharmaceutically acceptable salts thereof.

## Description

### Technical Field

This invention is related to pharmaceutical combination of betahistine or pharmaceutically acceptable salts thereof and trimetazidine or pharmaceutically acceptable salts thereof.

### Background Art

EP 1 108 424 B (LES LABORATOIRES SERVIER) 20.06.2001 and EP 0 673 649 B (LES LABORATOIRES SERVIER) 27.09.1995 claim prolonged release of trimetazidine or a pharmaceutically acceptable salt thereof to be used in the treatment of vertigo.

EP 1195160 A (USV LTD.) 10.04.2002 discloses a pharmaceutical composition for sustained release of trimetazidine dihydrochloride to be used in the treatment of vertigo.

MARTINI , A., et al. Trimetazidine versus betahistine in Ménière's disease. A double blind method. Annales d'oto-laryngologie et de chirurgie cervico faciale : bulletin de la Société d'oto-laryngologie des hôpitaux de Paris. 1990, vol.107, no.1, p.20-27. shows up that the efficacy and acceptability of trimetazidine in the treatment of Meniere's disease are compared with betahistine and trimetazidine is found to be significantly more effective than betahistine.

### Summary of invention

The invention relates to a combination of:
1. betahistine or a pharmaceutically acceptable salt thereof and
2. trimetazidine or a pharmaceutically acceptable salt thereof and
3. preferably an excipient or mixtures of excipients.

Said combination is used for the manufacture of a medicament for the treatment or prevention of:
1. Ménière's disease and/or,
2. Vertigo, tinnitus and hearing loss associated with Ménière's syndrome,

### Description of embodiments

Betahistine dihydrochloride (betahistine) is a histamine (H)I ,H2-receptor agonist and H3-receptor antagonist with antivertigo effect which improves the microcirculation in the labyrinth which reduces endolymphatic pressure.

Trimetazidine dihydrochloride (trimetazidine) is an anti-ischemic (anti-anginal) metabolic agent which selectively inhibits the activity of the final enzyme of the fatty acid β-oxidation pathway, long-chain 3-ketoacyl-coenzyme A thiolase (3-KAT). It is a cellular acting as anti-ischaemic agent which inhibits the anaerobic glycolysis and fatty acid metabolism, thus allowing only aerobic glycolysis. This action helps to restore the energy balance in the cell. It inhibits acidosis and free radical accumulation in the cell. All these action help the cell to restore the normal ionic and metabolic balance.

Trimetazidine is also employed as a symptomatic treatment of vertigo but its mechanism of action is yet to be defined.

In accordance with this invention an embodiment is invented for the manufacture of a medicament for the treatment or prevention of Ménière's disease and/or vertigo, tinnitus and hearing loss associated with Ménière's syndrome, wherein pharmaceutically effective amount of trimetazidine and betahistine are used in a fixed dosage combination or in a kit.

In another aspect of this invention, in order to enhance the patient compliance in the treatment of Ménière's disease and/or vertigo, tinnitus and hearing loss associated with Ménière's syndrome, the fixed dosage compositions of this invention are also presented. Accordingly, patients swallow only one newly invented pharmaceutical dosage form of combination of trimetazidine and betahistine.

In another aspect of this invention, patients may sequentially or simultaneously swallow separated tablets of trimetazidine and betahistine as parts of a kit.

This invention is directed to

(i) betahistine or a pharmaceutically acceptable salt thereof and

(ii) trimetazidine or a pharmaceutically acceptable salt thereof and

(iii) optionally an excipient or mixture of excipients.

In this invention the term of "betahistine" and the term of "trimetazidine" cover pharmaceutically acceptable bases, salts, isomers, racemates, racemic mixtures, individual diasteriomers, enantiomers. Furthermore, some of the crystalline forms for compounds of the present invention may exist as polymorphs and as such are intended to be included in the present invention. In addition, some of the compounds of the invention may form solvates with water or common organic solvents. Such solvates and hydrates, as well as anhydrous compositions, are encompassed within the scope of this invention.

The term "composition" or "combination" covers each other and can be used instead of other one.

All forms and mixtures thereof are included within the scope of this invention.

According to preferred embodiment of the invention is:

(i) betahistine or a pharmaceutically acceptable salt thereof and

(ii) trimetazidine or a pharmaceutically acceptable salt thereof and

(iii) optionally an excipient or mixture of excipients for the manufacture of a medicament for the treatment or prevention of:
1. Ménière's disease and/or,
2. Vertigo, tinnitus and hearing loss associated with Ménière's syndrome,

In another aspect this invention, betahistine is present in an amount of from 4 mg to 300 mg, preferably in an amount of from 6 mg to 30 mg and most preferably 24 mg and trimetazidine is present in an amount of from 10 mg to 80 mg, preferably in an amount of from 15 mg to 60 mg and most preferably 20 mg. Combinations may be for instance: 8 mg betahistine dihydrochloride and 20 mg trimetazidine dihydrochloride or: 16 mg betahistine dihydrochloride and 20 mg trimetazidine dihydrochloride or: 24 mg betahistine dihydrochloride and 20 mg trimetazidine dihydrochloride.

Preferably combination is administered two or three times daily. For instance 24 mg betahistine dihydrochloride and 20 mg trimetazidine dihydrochloride combination is used as b.i.d, 8 mg betahistine dihydrochloride and 20 mg trimetazidine dihydrochloride combination is used as t.i.d.

Preferred salt of betahistine is dihydrochloride and preferred salt of trimetazidine is dihydrochloride.

The pharmaceutical compositions according to the invention can be prepared for enteral, such as oral or rectal, and parenteral administration to mammals (warm-blooded animals), including human being.

For the manufacture of solid dosage forms, for example, the active ingredients may be mixed with the excipients and/or additives and granulated in a wet or dry process. Direct compression, melt granulation, melt congealing, extrusion process and such processes can be applied. Granules or powder can be filled directly into capsules or compressed into tablet cores.

These are prepared in a manner for example by means of mixing, granulating, dissolving, lyophilizing processes.

The pharmaceutical preparations contain, for example, from about 10 % to about 90 % of the active ingredients.

The components of a pharmaceutical combination can be dosed independently or by use of different fixed combinations. In other words administration may take place in the form of one fixed combination with two or more active ingredients, or by simultaneously administering two or more active ingredients that are formulated independently.

As a yet another aspect, this invention encompasses a kit or commercial package comprising a plurality of separate containers, wherein at least one container contains combination of betahistine and trimetazidine or a pharmaceutically acceptable salts thereof and at least one different container contains betahistine or trimetazidine or pharmaceutically acceptable salts thereof.

In accordance with this invention the kit comprising (i) therapeutically effective amount betahistine and optionally an acceptable carrier or diluent in a unit dosage form and (ii) therapeutically effective amount of trimetazidine and an acceptable carrier or diluent in a unit dosage form and (iii) a container.

According to this invention, each unit dosage form is discrete dosage forms that are packaged together. The kits of the invention can include container as separate compositions such as a divided bottle or a divided packet. Separate unit dosage forms can also be contained in undivided container. The pharmaceutical kits can include instructions as a separate sheet or optionally printed on the containers.

The pharmaceutical combination can concurrently, separately or sequentially be administered with an instruction.

It is preferred that betahistine and trimetazidine be co-administered concurrently on three times-per-day dosing schedule. However, varying dosing schedules, such as b.i.d, are also encompassed herein.

A single oral dosage formulation comprised of both betahistine and trimetazidine is preferred. The single dosage formulation will provide convenience for the patient, where patients who needs multiple medications.

Total % quantity of drug substances, Trimetazidine DiHCl and Betahistine DiHCl, or pharmaceutically acceptable salts thereof may vary between 1 - 90% of total weight of pharmaceutical dosage form.

Preferably, total % quantity of drug substances or pharmaceutically acceptable salts thereof varies between 10 - 50% of total weight of pharmaceutical dosage form.

Total % quantity of diluent(s) may vary between 5 - 90% of total weight of pharmaceutical dosage form.

Preferably, total % quantity of diluent(s) varies between 20 - 70% of total weight of pharmaceutical dosage form.

Total % quantity of binder(s) may vary between 2 - 30% of total weight of pharmaceutical dosage form.

Preferably, total % quantity of binder(s) varies between 4 - 20% of total weight of pharmaceutical dosage form.

Total % quantity of disintegrant(s) may vary between 1 - 25% of total weight of pharmaceutical dosage form.

Preferably, total % quantity of disintegrant(s) varies between 3 - 20% of total weight of pharmaceutical dosage form.

Total % quantity of lubricant(s) may vary between 0.1 - 2% of total weight of pharmaceutical dosage form.

Preferably, total % quantity of lubricant(s) varies between 0.3 - 1.5% of total weight of pharmaceutical dosage form.

Total % quantity of flow enhancer(s) may vary between 0.1 - 2% of total weight of pharmaceutical dosage form.

Preferably, total % quantity of flow enhancer(s) varies between 0.5 - 2% of total weight of pharmaceutical dosage form.

Total % quantity of effervescent agent(s) may vary between 10 - 90% of total weight of pharmaceutical dosage form.

Preferably, total % quantity of effervescent agent(s) varies between 40 - 80% of total weight of pharmaceutical dosage form.

Total % quantity of patient compliance enhancer(s) (including sweeteners, flavours, flavour enhancer, cooling agent, taste masking agent etc.) may vary between 0.5 - 90% of total weight of pharmaceutical dosage form.

Preferably, total % quantity of patient compliance enhancer(s) (including sweeteners, flavours, flavour enhancer, cooling agent, taste masking agent etc.) varies between 2 - 50% of total weight of pharmaceutical dosage form.

Total % quantity of suspending agent(s) may vary between 2 - 90% of total weight of pharmaceutical dosage form.

Preferably, total % quantity of suspending agent(s) varies between 4 - 70% of total weight of pharmaceutical dosage form.

Total % quantity of viscosity increasing agent(s) may vary between 0.1 - 10% of total weight of pharmaceutical dosage form.

Preferably, total % quantity of viscosity increasing agent(s) varies between 0.2 - 5% of total weight of pharmaceutical dosage form.

Total % quantity of buffering agent(s) may vary between 0.01 - 3% of total weight of pharmaceutical dosage form.

Preferably, total % quantity of buffering agent(s) varies between 0.1 - 2% of total weight of pharmaceutical dosage form.

Total % quantity of preservative agent(s) may vary between 0.01 - 4% of total weight of pharmaceutical dosage form.

Preferably, total % quantity of preservative agent(s) varies between 0.1 - 3% of total weight of pharmaceutical dosage form.

Ratio of quantity of active substances or pharmaceutically acceptable salts thereof to quantity of basic effervescent agent(s) varies from 1:10 to 10:1.

Preferably, ratio of quantity of active substances or pharmaceutically acceptable salts thereof to quantity of basic effervescent agent(s) varies from 1:4 to 4:1.

Ratio of quantity of active substances or pharmaceutically acceptable salts thereof to quantity of acidic effervescent agent(s) varies from 1:8 to 8:1.

Preferably, ratio of quantity of active substances or pharmaceutically acceptable salts thereof to quantity of acidic effervescent agent(s) varies from 1:2 to 2:1.

Ratio of quantity of active substances or pharmaceutically acceptable salts thereof to quantity of patient compliance enhancer(s) (including sweeteners, flavours, flavour enhancer, cooling agent, taste masking agent etc.) varies from 1:1 to 20:1.

Preferably, ratio of quantity of active substances or pharmaceutically acceptable salts thereof to quantity of patient compliance enhancer(s) (including sweeteners, flavours, flavour enhancer, cooling agent, taste masking agent etc.) varies from 3:1 to 15:1.

Ratio of quantity of active substances or pharmaceutically acceptable salts thereof to quantity of viscosity increasing agent(s) varies from 1:100 to 100:1.

Preferably, ratio of quantity of active substances or pharmaceutically acceptable salts thereof to quantity of viscosity increasing agent(s) varies from 1:70 to 70:1.

Ratio of quantity of active substances or pharmaceutically acceptable salts thereof to quantity of diluent(s) varies from 1:10 to 10:1.

Preferably, ratio of quantity of active substances or pharmaceutically acceptable salts thereof to quantity of diluent(s) varies from 1:5 to 5:1.

Ratio of quantity of active substances or pharmaceutically acceptable salts thereof to quantity of buffering agent(s) varies from 1:200 to 1:2.

Preferably, ratio of quantity of active substances or pharmaceutically acceptable salts thereof to quantity of buffering agent(s) varies from 1:100 to 1:10.

Ratio of quantity of active substances or pharmaceutically acceptable salts thereof to quantity of disintegrant(s) varies from 1:10 to 10:1.

Preferably, ratio of quantity of active substances or pharmaceutically acceptable salts thereof to quantity of disintegrant(s) varies from 1:4 to 8:1.

Pharmaceutical combination may be prepared using methods as below without limiting scope of this invention;
- Both active agents are separately granulated and then granulates are combined or,
- One active agent is granulated and then granulate is combined with non granulated active agent or,
- Both active pharmaceutical ingredients are granulated together or,
- One active agent is dissolved alone or with one or more excipients and then other active agent alone or with one or more excipients are coated with said solution
- One active agent is dispersed alone or with one or more excipient and then other active agent alone or with one or more excipients are coated or adhered with said dispersion

Said solution or dispersion, mention in previous paragraph comprises ;

(i) an inorganic acid or mixtures of inorganic acids or,

(ii) an organic acid or mixtures of organic acids or,

(iii) mixtures of organic acid or organic acids and inorganic acid or inorganic acids and

(v) an organic solvent or mixtures of organic solvents and

(vi) optionally de-ionized water and,

(vii) optionally binder or binders.

Organic solvents used in preparation of solution or dispersion are but not limited to, alcohols such as methanol, ethanol, propyl alcohol, isopropyl alcohol, 2-methyl-1-propanol etc. ; ketones such as acetone, methyl ethyl ketone, methyl isobutyl ketone etc. ; esters such as methyl acetate, ethyl acetate, propyl acetate, isopropyl acetate, ethyl formate etc. ; hydrocarbons such as heptane ; halogenated hydrocarbons such as dichloromethane.

Organic acids are but not limited to formic acid, acetic acid, fumaric acid, tartaric acid, maleic acid, citric acid, succinic acid, malic acid, methanesulfonic acid,p-toluenesulfonic acid .

Inorganic acid is hydrochloric acid in the form of solution in water.

A further object of the instant invention involves following preparations :
- Betahistine and trimetazidine are separately granulated and then they are separately compressed into mini tablets and filled into capsule altogether or
- Betahistine and trimetazidine are separately granulated and then they are separately compressed into tablet and filled into capsule or
- One of the active agents, betahistine or trimetazidine, is granulated and compressed into tablet or mini-tablets and then combined with granules of other active agent, betahistine or trimetazidine , into capsule or
- Betahistine and trimetazidine are separately granulated and then directly filled into capsule without tabletting or
- One of the active agents, betahistine or trimetazidine, is prepared through direct compression into tablet or mini-tablets and then combined with granules of other active agent, betahistine or trimetazidine, into capsule.

Pharmaceutical preparations according to the invention are, for example, tablet, bilayer tablet, tablet in tablet, sugar coated tablet, capsule, tablet in capsule, inlay tablet, pellets in capsule, suppositories, powders, granules, granules in capsule, effervescent tablet, readily dissolved granule, effervescent granules, effervescent pellets, effervescent dry powder, readily dispersible granule and other dosage forms suitable for oral administration. Liquid preparations may be such as solutions, ampoules, elixir, intravenous and intramuscular forms, suspensions or emulsions.

In an embodiment, the present invention provides tablet-in-tablet compositions comprising: a) a core tablet comprising: trimetazidine or pharmaceutically acceptable salts thereof and a pharmaceutically acceptable carrier or carriers b) a compressed outer tablet layer comprises: betahistine or pharmaceutically acceptable salts thereof and a pharmaceutically acceptable carrier or carriers. Alternatively, tablet-in-tablet compositions comprising: a) a core tablet comprising: betahistine or pharmaceutically acceptable salts thereof and a pharmaceutically acceptable carrier or carriers b) a compressed outer tablet layer comprising: trimetazidine or pharmaceutically acceptable salts thereof and a pharmaceutically acceptable carrier or carriers.

In another embodiment, this invention embraces bilayer tablet. Bilayer tablet comprises; a) a first layer containing trimetazidine or pharmaceutically acceptable salts thereof and a pharmaceutically acceptable carrier or carriers and b) a second layer containing betahistine or pharmaceutically acceptable salts thereof and a pharmaceutically acceptable carrier or carriers. Alternatively, bilayer tablet comprises a) a first layer containing betahistine or pharmaceutically acceptable salts thereof and a pharmaceutically acceptable carrier or carriers and b) a second layer containing trimetazidine or pharmaceutically acceptable salts thereof and a pharmaceutically acceptable carrier or carriers.

The expression bilayer is not limited to two layers also multilayer tablets are meant with this expression.

The first and the second layers of bilayer tablet can also be arranged side-by-side.

In still another embodiment, this invention delineates an inlay tablet. The inlay tablet comprises that inner tablet is encompassed by an outer tablet wherein at least a portion of one surface of the inner tablet is not surrounded by outer tablet. The inner tablet comprises betahistine and the outer tablet comprises trimetazidine or vice versa. An excipient or mixture of excipients can be used.

In still another aspect, this invention embraces capsule in capsule formulations wherein smaller size capsule is encapsulated into a larger capsule. Capsule-in-capsule consists of an external capsule and internal capsule (inner capsule) located therein. It is preferred that smaller size capsule is filled with trimetazidine and optionally excipients and larger capsule is filled with betahistine and optionally excipients. Capsule in capsule operations can be performed via Modu-C (HarroHofliger).

Another aspect of this invention is to provide spray dried powders of trimetazidine or betahistine or mixtures of betahistine and trimetazidine. To prepare slurry or solution for spray drying, suitable excipients such as binders and disintegrants may be combined with the active agents. The slurry or solution of active agents can be directly spray dried or they can be combined with one or more excipients before spray drying. The slurry or solution can be spray dried by the use of conventional spray drying apparatuses like vertical spray dryer or fluid spray dryer. For instance Buchi Mini Spray Dryer B-290 can be used.

In still another embodiment of this invention is directed to effervescent tablet, effervescent granules, effervescent pellets and effervescent dry powder. Effervescent forms use of a chemical reaction between a soluble acid and alkali metal carbonate with the help of water to make CO₂ gas, which can give a fizzy taste in the mouth. Any conventional effervescent agent, for example a mixture of a substance which on dissolution in water forms an acid reacting solution, and a pharmaceutically acceptable carbonate can be used as effervescent agent. The acid component of the composition can be any suitable acid for effervescent compositions. Typically, the acid is organic or mineral acid that is safe and approved for pharmaceutical and/or nutritional purposes and which provides effective and rapid effervescent disintegration upon contact with water and the alkaline effervescent compound. The acid may be selected from food acids, acid anhydrides and acid salts.

As a yet further aspect, prepared granulates can be filled into capsules. Capsules can be consisted of separated compartments and each active ingredient and it's granulates can be filled into each compartment.

According to this invention, combination includes one or more pharmaceutically acceptable excipients, carriers, or diluents. In formulations, surfactants, diluents, sweeteners, disintegrants, direct compression agents, acids for effervescent forms, binders, lubricants, glidants, colorants, alkaline effervescing agents, flavors and mixtures thereof can be used.

Diluents are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst™, mixtures thereof and the like.

Glidants are, but not limited to, silicon dioxide, colloidal silicon dioxide, calcium silicate, magnesium silicate, magnesium trisilicate, talc, starch, mixtures thereof and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like .

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose,lower alkylsubstituted hydroxypropyl cellulose, polacrilin potassium, starch, pregelatinized starch, sodium alginate, mixtures thereof or whatsoever.

Direct compression agents are, but not limited to, pregelatinized starch, polyvinylpyrrolidone, methylcellulose, microcrystalline cellulose, sucrose, lactose, dextrose, sorbitol, mannitol, lactitol,xylitol, modified calcium salt, granulated corn starch, modified rice starch, compressible sugars such as Destab^{™}, dextrates such as Emdex ® dicalcium phosphate, hydroxypropylcellulose , methylcellulose , hydroxypropylmethylcellulose, polyethylene glycol, amylose,anhydrous calcium hydrogen phosphate, calcium sulphate,tribasic calcium phosphate, dibasic calcium phosphate, low-crystallinity powdered cellulose,silicified microcrystalline cellulose,chitin,chitosan hydrochloride,copovidone,croscarmellose sodium,dextrose, anhydrous lactose,anhydrous alpha lactose,anhydrous beta lactose,agglomerated lactose, spray-dried lactose, maltodextrin, mixtures thereof and the like.

Acids are, but not limited to, citric acid, tartaric acid, ascorbic acid, fumaric acid, malic acid, adipic and succinic acid, acetyl salicylic acid, nicotinic acid, citric anhydride, succinic anhyride, glutamic anhydride, sodium hydrogen phosphate, disodium dihydrogen pyrophospate, acid citrate salts, aminoacid hydrochlorides, mixtures thereof and the like.

Alkaline effervescing agents are, but not limited to, sodium bicarbonate, sodium carbonate, potassium bicarbonate and potassium carbonate, sodium sesquicarbonate, sodium glycine carbonate, L-lysine carbonate, arginine carbonate, amorphous calcium carbonate, mixtures thereof and the like.

Flavors are, but not limited to, cinnamon oil,essence of apple, essence of pear, essence of peach, essence of grape, essence of strawberry, essence of raspberry, essence of cherry,essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehyde, mixtures thereof and the like.

Sweeteners are but not limited to, corn syrup, dextrose, cyclamate, invert sugar, fructose, saccharin, aspartame, acesulfame-K, Stevia rebaudiana, sucralose, sorbitol, mannitol, xylitol, mixtures thereof and the like.

### Example 1 (Unit Formula: Single Tablet)

Trimetazidine DiHCl + Betahistine DiHCl Single Tablet:

**Table 1**

| | **mg/tablet** | **% (w/w)** | **Function** |
|---|---|---|---|
| **Active Substances** | | | |
| Trimetazidine DiHCl | 20.00 | 10.00 | Drug Substance |
| Betahistine DiHCl | 24.00 | 12.00 | Drug Substance |

| **Excipients** | | | |
|---|---|---|---|
| Microcrystalline Cellulose (Avicel PH 101) | 26.00 | 13.00 | Diluent |
| Microcrystalline Cellulose (Avicel PH 102) | 30.00 | 15.00 | Diluent/ Compression Aid |
| Mannitol | 65.00 | 32.50 | Diluent |
| Pregelatinized Starch (Starch 1500) | 24.00 | 12.00 | Disintegrant |
| Povidone (K-30) | 9.00 | 4.50 | Binder |
| Magnesium Stearate | 2.00 | 1.00 | Lubricant |
| **Total Core Tablet Weight** | **200.00** | **100.00** | |

Core tablets are optionally coated with dispersion of film coating material either dispersed in water or water and alcohol mixture or organic solvent. Total film coating applied may vary 1 - 5 % (w/w) of core tablet. Preferred applied film coating is 3 % (w/w) of core tablet.

### Example 2 (Manufacturing Process of Example 1)

Manufacturing Process of Example 1 :
1. Trimetazidine DiHCl, Betahistine DiHCl, Avicel PH 101, 1/3 of Starch 1500 and Mannitol are mixed.
2. Povidone K-30 is dissolved in water.
3. Powder mixture in Step 1 is granulated with solution in Step 2 using high shear mixer, Collette UltimaGral Granulator.
4. Wet granules are dried using Fluid Bed Drier, Aeromatic MP3, until moisture content is less than 4%.
5. Dry granules are mixed with remaining Starch 1500 and Avicel PH 102.
6. Magnesium Stearate is added and mixed.
7. Core tablets are compressed with suitable punches having average target weight 200 mg/tablet.
8. Core tablets are optionally film coated. Opadry Red Y-5-1842 can be used as film coating agent.
9. 12% (w/w) dispersion of Opadry Red Y-5-1842 is prepared in water.
10. Core tablets are film coated with above dispersion using GS Perfima Film Coating Machine until desired film coating applied.

### Example 3 (Unit Formula: Single Tablet)

Trimetazidine DiHCl + Betahistine DiHCl Single Tablet:

**Table 2**

| | **mg/tablet** | **% (w/w)** | **Function** |
|---|---|---|---|
| **Active Substances** | | | |
| Trimetazidine DiHCl | 20.00 | 10.00 | Drug Substance |
| Betahistine DiHCl | 24.00 | 12.00 | Drug Substance |

| **Excipients** | | | |
|---|---|---|---|
| Microcrystalline Cellulose (Avicel PH 102) | 32.00 | 16.00 | Diluent/ Compression Aid |
| Mannitol | 102.00 | 51.00 | Diluent |
| Crospovidone | 8.00 | 4.00 | Disintegrant |
| Povidone (K-30) | 12.00 | 6.00 | Binder |
| Magnesium Stearate | 2.00 | 1.00 | Lubricant |
| **Total Core Tablet Weight** | **200.00** | **100.00** | |

Core tablets are optionally coated with dispersion of film coating material either dispersed in water or water and alcohol mixture or organic solvent. Total film coating applied may vary 1 - 5 % (w/w) of core tablet. Preferred applied film coating is 3 % (w/w) of core tablet.

### Example 4 (Manufacturing Process of Example 3)

Manufacturing Process of Example 3 by Spray Drying :
1. Trimetazidine DiHCl, Betahistine DiHCl and Mannitol are mixed in Fluid Bed Granulator (Vector VFC-30X).
2. Povidone K-30 is dissolved in water:alcohol (60:40) mixture.
3. Powder mixture in Step 1 is spray granulated (simultaneous granulation and drying) with solution in Step 2.
4. Drying is continued until moisture content is less than 4%.
5. Dry granules are mixed with Crospovidone and Avicel PH 102.
6. Magnesium Stearate is added and mixed.
7. Core tablets are compressed with suitable punches having average target weight 200 mg/tablet.
8. Core tablets are optionally film coated. Opadry II 85F16122 Red can be used as film coating agent.
9. 18% (w/w) dispersion of Opadry II 85F16122 Red is prepared in water+alcohol (70:30) mixture.
10. Core tablets are film coated with above dispersion using perforated coating machine, Vector VHC-1355 Hi-Coater, until desired film coating applied.

### Example 5 Tablet-in-tablet: Unit Formula)

Trimetazidine DiHCl + Betahistine DiHCl Tablet in Tablet:

**Table 3**

| | **mg/tablet** | **% (w/w)*** | **Function** |
|---|---|---|---|
| **Trimetazidine Tablet** | | | |
| Trimetazidine DiHCl | 20.00 | 6.67 | Drug Substance |
| Microcrystalline Cellulose (Avicel PH 102) | 54.60 | 18.20 | Diluent/ Compression Aid |
| Povidone (K-30) | 8.50 | 2.83 | Dry Binder |
| Crospovidone | 6.00 | 2.00 | Disintegrant |
| Magnesium Stearate | 0.90 | 0.30 | Lubricant |
| **Total Trimetazidine Tablet Weight** | **90.00** | | |

| **Betahistine Granule** | | | |
|---|---|---|---|
| Betahistine DiHCl | 24.00 | 8.00 | Drug Substance |
| Mannitol | 50.50 | 16.83 | Diluent |
| Pregelatinized Starch (Starch 1500) | 26.00 | 8.67 | Dry Binder |
| Croscarmellose Sodium | 19.50 | 6.50 | Disintegrant |
| Microcrystalline Cellulose (Avicel PH 102) | 84.00 | 28.00 | Diluent / Compression Aid |
| Colloidal Silicon Dioxide (Aerosil 200) | 3.50 | 1.17 | Flow Enhancer |
| Magnesium Stearate | 2.50 | 0.83 | Lubricant |
| **Total Betahistine Granule Weight** | **210.00** | | |
| **Total Core Tablet Weight** | **300.00** | | |

* % values are calculated according to final core tablet weight.

Core tablets are optionally coated with dispersion of film coating material either dispersed in water or water and alcohol mixture or organic solvent. Total film coating applied may vary 1 - 5 % (w/w) of core tablet. Preferred applied film coating is 3 % (w/w) of core tablet.

### Example 6 (Manufacturing Process of Example 5)

Manufacturing Process of Example 5:
1. Trimetazidine DiHCl, Avicel PH 102 PVP K-30 and Crospovidone are mixed.
2. Powder mixture in Step 1 is sieved through 0.6 mm sieve.
3. Magnesium Stearate is added and mixed.
4. Trimetazidine Tablets are compressed with suitable punches having average target weight of 90 mg/tablet.
5. Betahistine DiHCl, Mannitol, Starch 1500, Croscarmellose Sodium and Aerosil 200 are mixed.
6. Powder mixture in Step 5 is sieved through 0.5 mm sieve and mixed again.
7. Avicel PH 102 is added to powder mixture in Step 6 and mixed.
8. Magnesium Stearate is added to powder mixture in Step 7, mixed and Betahistine Granules ready for compression is prepared.
9. Using IMA S250 ZS/M Tablet Press Machine, which is capable of compressing tablet-in-tablet, Trimetazidine Tablets are compressed with Betahistine Granules.
10. Average target weight of final core tablets is 300 mg/tablet.

### Example 7 (Bilayer Tablet)

Trimetazidine DiHCl + Betahistine DiHCl Double Layer Tablet:

**Table 4**

| | **mg/tablet** | **% (w/w)*** | **Function** |
|---|---|---|---|
| **Trimetazidine Layer** | | | |
| Trimetazidine DiHCl | 20.00 | 6.67 | Drug Substance |
| Microcrystalline Cellulose (Avicel PH 101) | 42.00 | 14.00 | Diluent |
| Povidone (K-30) | 9.50 | 3.17 | Binder |
| Pregelatinized Starch (Starch 1500) | 28.00 | 9.33 | Disintegrant |
| Magnesium Stearate | 0.50 | 0.17 | Lubricant |
| **Total Trimetazidine Layer Weight** | **100.00** | | |

| **Betahistine Layer** | | | |
|---|---|---|---|
| Betahistine DiHCl | 24.00 | 8.00 | Drug Substance |
| Mannitol | 32.50 | 10.83 | Diluent |
| Pregelatinized Starch (Starch 1500) | 45.50 | 15.17 | Dry Binder |
| Crospovidone | 14.00 | 4.67 | Disintegrant |
| Microcrystalline Cellulose (Avicel PH 102) | 76.00 | 25.32 | Diluent / Compression Aid |
| Colloidal Silicon Dioxide (Aerosil 200) | 4.50 | 1.50 | Flow Enhancer |
| Magnesium Stearate | 3.50 | 1.17 | Lubricant |
| **Total Betahistine Layer Weight** | **200.00** | | |
| **Total Core Tablet Weight** | **300.00** | | |

* % values are calculated according to final core tablet weight.

Core tablets are optionally coated with dispersion of film coating material either dispersed in water or water and alcohol mixture or organic solvent. Total film coating applied may vary 1 - 5 % (w/w) of core tablet. Preferred applied film coating is 3 % (w/w) of core tablet.

### Example 8 (Manufacturing Process of Example 7)

Manufacturing Process of Example 7:
1. Trimetazidine DiHCl and Avicel PH 101 are mixed.
2. PVP K-30 is dissolved in water.
3. Powder mixture in Step 1 is spray granulated with solution in Step2.
4. Granules are dried until moisture content is less than 6% (w/w).
5. Dry granules are sieved through 0.6 mm sieve and mixed again.
6. Magnesium Stearate is added to powder mixture in Step 5, mixed and Trimetazidine Layer ready for compression is prepared.
7. Betahistine DiHCl, Mannitol, Starch 1500 and Aerosil 200 are mixed.
8. Powder mixture in Step 7 is sieved through 0.5 mm sieve and mixed again.
9. Crospovidone and Avicel PH 102 are added to powder mixture in Step 8 and mixed.
10. Magnesium Stearate is added, mixed and Betahistine Layer ready for compression is prepared.
11. Double layer tablets are compressed from Trimetazidine Layer and Betahistine Layer using Fette 102i Tablet Press Machine.
12. Average target weight of final core tablets is 300 mg/tablet.

### Example 9 (Capsule)

Trimetazidine DiHCl + Betahistine DiHCl Capsule:

**Table 5**

| | **mg/capsule** | **% (w/w)** | **Function** |
|---|---|---|---|
| **Active Substances** | | | |
| Trimetazidine DiHCl | 20.00 | 6.67 | Drug Substance |
| Betahistine DiHCl | 24.00 | 8.00 | Drug Substance |

| Excipients | | | |
|---|---|---|---|
| Mannitol | 171.00 | 57.00 | Diluent |
| Copovidone | 43.00 | 14.33 | Dry Binder |
| Maize Starch (Dried) | 34.00 | 11.33 | Disintegrant |
| Colloidal Silicon Dioxide (Aerosil 200) | 3.50 | 1.17 | Flow Enhancer |
| Magnesium Stearate | 4.50 | 1.50 | Lubricant |
| **Total Powder Weight in Capsule** | **300.00** | **100.00** | |

### Example 10 (Manufacturing Process of Example 9)

Manufacturing Process of Example 9:
1. Maize Starch is dried until loss on drying is less than 5% (w/w).
2. Trimetazidine DiHCl, Betahistine DiHCl, Mannitol, Copovidone and Maize Starch (Dried) are mixed.
3. Aerosil 200 is added to power mixture in Step 2, sieved through 0.8 mm sieve and mixed again.
4. Magnesium Stearate is added and mixed.
5. Powder mixture in Step 4 is filled into hard gelatin capsules having average target powder weight of 300 mg/capsule.

### Example 11 Trimetazidine DiHCl + Betahistine DiHCl Direct Compression Tablet)

Trimetazidine DiHCl + Betahistine DiHCl Direct Compression Tablet:

**Table 6**

| | **mg/capsule** | **% (w/w)** | **Function** |
|---|---|---|---|
| **Active Substances** | | | |
| Trimetazidine DiHCl | 20.00 | 5.71 | Drug Substance |
| Betahistine DiHCl | 24.00 | 6.86 | Drug Substance |

| **Excipients** | | | |
|---|---|---|---|
| Microcrystalline Cellulose (Avicel PH 102) | 140.00 | 40.00 | Diluent/ Compression Aid |
| Pregelatinized Starch (Starch 1500) | 44.00 | 12.57 | Dry Binder |
| Crospovidone | 29.50 | 8.43 | Disintegrant |
| Mannitol | 85.00 | 24.29 | Diluent |
| Colloidal Silicon Dioxide (Aerosil 200) | 4.00 | 1.14 | Flow Enhancer |
| Magnesium Stearate | 3.50 | 1.00 | Lubricant |
| **Total Powder Weight in Capsule** | **350.00** | **100.00** | |

Core tablets are optionally coated with dispersion of film coating material either dispersed in water or water and alcohol mixture or organic solvent. Total film coating applied may vary 1 - 5 % (w/w) of core tablet. Preferred applied film coating is 3 % (w/w) of core tablet.

### Example 12 (Manufacturing Process of Example 11)

Manufacturing Process of Example 11:
1. Trimetazidine DiHCl, Betahistine DiHCl, Starch 1500, Aerosil 200 and Mannitol are mixed.
2. Powder mixture in Step 1 is sieved through 0.6 mm sieve and mixed again.
3. Avicel PH 102 and Crospovidone are added and mixed.
4. Magnesium Stearate is added and mixed.
5. Powder mixture in Step 4 is compressed with suitable punches having average target tablet weight of 350 mg/tablet.

### Example 13 Trimetazidine DiHCl + Betahistine DiHCl Dry Granulation Tablet)

Unit Formula:

**Table 7**

| | **mg/tablet** | **% (w/w)** | **Function** |
|---|---|---|---|
| **Active Substances** | | | |
| Trimetazidine DiHCl | 20.00 | 20.00 | Drug Substance |
| Betahistine DiHCl | 24.00 | 24.00 | Drug Substance |

| **Excipients** | | | |
|---|---|---|---|
| Microcrystalline Cellulose (Avicel PH 102) | 32.90 | 32.90 | Diluent |
| Pregelatinized Starch (Starch 1500) | 16.00 | 16.00 | Dry Binder |
| Crospovidone | 6.50 | 6.50 | Disintegrant |
| Magnesium Stearate | 0.60 | 0.60 | Lubricant |
| **Total Core Tablet Weight** | **100.00** | **100.00** | |

Core tablets are optionally coated with dispersion of film coating material either dispersed in water or water and alcohol mixture or organic solvent. Total film coating applied may vary 1 - 5 % (w/w) of core tablet. Preferred applied film coating is 3 % (w/w) of core tablet.

### Example 14 (Manufacturing Process of Example 13)

Manufacturing Process:
1. Trimetazidine DiHCl, Betahistine DiHCl, Avicel PH 102, Starch 1500 and 2/3 of Crospovidone are mixed.
2. Powder mixture in Step 1 is dry granulated with roller compactor (Powtec) or slugged with suitable punches.
3. Compacted granules or slug tablets are crushed and sieved through 0.8 mm sieve.
4. Powder mixture in step 3 is mixed.
5. Remaining Crospovidone is added and mixed.
6. Magnesium Stearate is added and mixed.
7. Powder mixture in Step 6 is compressed with suitable punches having average target tablet weight of 100 mg/tablet.

### Example 15 Trimetazidine DiHCl + Betahistine DiHCl Tablet in Capsule)

Unit Formula:

**Table 8**

| | **mg/capsule** | **% (w/w)*** | **Function** |
|---|---|---|---|
| **Trimetazidine Tablet** | | | |
| Trimetazidine DiHCl | 20.00 | 6.67 | Drug Substance |
| Microcrystalline Cellulose (Avicel PH 102) | 61.30 | 20.43 | Diluent/ Compression Aid |
| Pregelatinized Starch (Starch 1500) | 11.50 | 3.83 | Binder |
| Crospovidone | 6.00 | 2.00 | Disintegrant |
| Magnesium Stearate | 1.20 | 0.40 | Lubricant |
| **Total Trimetazidine Tablet Weight** | **100.00** | | |

| **Betahistine Granule** | | | |
|---|---|---|---|
| Betahistine DiHCl | 24.00 | 8.00 | Drug Substance |
| Mannitol | 48.50 | 16.17 | Diluent |
| Copovidone | 17.50 | 5.83 | Dry Binder |
| Maize Starch (Dried) | 22.00 | 7.33 | Disintegrant |
| Microcrystalline Cellulose (Avicel PH 102) | 85.50 | 28.51 | Diluent |
| Magnesium Stearate | 2.50 | 0.83 | Lubricant |
| **Total Betahistine Granule Weight** | **200.00** | | |
| **Total Weight Filled into Capsule** | **300.00** | | |

* % values are calculated according to final weight in capsule.

### Example 16 (Manufacturing Process of Example 15):

Manufacturing Process:
1. Trimetazidine DiHCl, ½ of Avicel PH 102 and ½ of Crospovidone are mixed.
2. Starch 1500 is dispersed in water.
3. Powder mixture in Step 1 is spray granulated with dispersion in Step 2.
4. Granules are dried until moisture is less than 4% (w/w).
5. Dry granules are sieved and mixed.
6. Remaining Avicel PH 102 and Crospovidone are added and mixed.
7. Magnesium Stearate is added and mixed.
8. Trimetazidine Tablets are compressed with suitable punches having average target weight of 100 mg/tablet.
9. Maize Starch is dried until loss on drying is less than 5% (w/w).
10. Betahistine DiHCl, Mannitol, Copovidone, Maize Starch (Dried) and Avicel PH 102 are mixed.
11. Magnesium Stearate is added, mixed and Betahistine Granules ready for encapsulation is prepared.
12. Using Zanasi 40E Capsule Filling Machine, which is capable of filling both tablet and powder into capsule, Trimetazidine Tablets and Betahistine Granules are filled into hard gelatin capsule.
13. Average target weight inside of capsules is 300 mg/capsule.

### Example 17 Trimetazidine DiHCl + Betahistine DiHCl Effervescent Tablet:

Unit Formula:

**Table 9**

| | **mg/tablet** | **% (w/w)** | **Function** |
|---|---|---|---|
| **Active Substances** | | | |
| Trimetazidine DiHCl | 20.00 | 1.67 | Drug Substance |
| Betahistine DiHCl | 24.00 | 2.00 | Drug Substance |

| **Excipients** | | | |
|---|---|---|---|
| Potassium Bicarbonate | 620.00 | 51.67 | Effervescent Agent (Basic) |
| Citric Acid Monohydrate | 290.00 | 24.17 | Effervescent Agent (Acidic) |
| Sucralose | 30.00 | 2.50 | Sweetener |
| Acesulfame Potassium | 35.00 | 2.92 | Sweetener |
| Maltodextrin | 133.00 | 11.07 | Diluent |
| Orange Flavour | 30.00 | 2.50 | Flavouring Agent |
| Talc | 18.00 | 1.50 | Lubricant |
| **Total Tablet Weight** | **1200.00** | **100.0** | |

### Example 18 (Manufacturing Process of Example 17)

Manufacturing Process:
1. Trimetazidine DiHCl, Betahistine DiHCl and Maltodextrin are mixed.
2. Potassium Bicarbonate, powder mixture in Step 1 and Citric Acid Monohydrate are mixed.
3. Sucralose, Acesulfame Potassium and Orange Flavour are dissolved/dispersed in water:alcohol (30:70) mixture.
4. Powder mixture in Step 2 is granulated with solution/dispersion prepared in Step 3 utilizing fluid bed granulator.
5. Granules are dried until moisture content is less than 1% (w/w).
6. Granules are sieved through 0.8 mm sieve.
7. Talc is added to dry granules and mixed.
8. Effervescent tablets are compressed with suitable punches having average target weight 1200 mg/tablet.

### Example 19 (Trimetazidine DiHCl + Betahistine DiHCl Readily Dissolved

### Granules

Unit Formula:

**Table 10**

| | **mg/package** | **% (w/w)** | **Function** |
|---|---|---|---|
| **Active Substances** | | | |
| Trimetazidine DiHCl | 20.00 | 5.00 | Drug Substance |
| Betahistine DiHCl | 24.00 | 6.00 | Drug Substance |

| **Excipients** | | | |
|---|---|---|---|
| Sucralose | 45.00 | 11.25 | Sweetener |
| Sodium Saccharin | 25.00 | 6.25 | Sweetener |
| Mannitol | 266.00 | 66.50 | Diluent/Sweetener |
| Mellon Flavour | 20.00 | 5.00 | Flavouring Agent |
| **Total Tablet Weight** | **400.00** | **100.0** | |

### Example 20 Manufacturing Process of Example 19)

Manufacturing Process:
1. Trimetazidine DiHCl, Betahistine DiHCl, Sucralose, Sodium Saccharin and Mellon Flavour are mixed.
2. Powder mixture in Step 1 is sieved through 0.4 mm sieve and mixed.
3. ½ of Mannitol, powder mixture in Step 2 and ½ of Mannitol are mixed.
4. Powder mixture in Step 3 is sieved through 0.6 mm sieve and mixed.
5. Powder mixture in Step 4 is filled into suitable packaging material, such as sachets, having average target powder weight of 400 mg/package.

### Example 21 Trimetazidine DiHCl + Betahistine DiHCl Readily Dispersed Granules)

Unit Formula:

**Table 11**

| | **mg/package** | **% (w/w)** | **Function** |
|---|---|---|---|
| **Active Substances** | | | |
| Trimetazidine DiHCl | 20.00 | 3.33 | Drug Substance |
| Betahistine DiHCl | 24.00 | 4.00 | Drug Substance |

| **Excipients** | | | |
|---|---|---|---|
| Sucralose | 35.00 | 5.83 | Sweetener |
| Acesulfame Potassium | 35.00 | 5.83 | Sweetener |
| Mannitol | 76.00 | 12.67 | Diluent/Sweetener |
| Carmellose Calcium | 370.00 | 61.67 | Suspending Agent |
| Citric Acid | 15.00 | 2.50 | Flavour Enhancer |
| Tutti Frutti Flavour | 25.00 | 4.17 | Flavouring Agent |
| **Total Tablet Weight** | **600.00** | **100.0** | |

### Example 22 (Manufacturing Process of Example 21)

Manufacturing Process:
1. Trimetazidine DiHCl, Betahistine DiHCl, Sucralose, Acesulfame Potassium, Mannitol, Citric Acid and Tutti Frutti Flavour are mixed.
2. Powder mixture in Step 1 is sieved through 0.6 mm sieve and mixed.
3. ½ of Carmellose Calcium, powder mixture in Step 2 and ½ of Carmellose Calcium are mixed.
4. Powder mixture in Step 3 is sieved through 0.8 mm sieve and mixed.
5. Powder mixture in Step 4 is filled into suitable packaging material, such as sachets, having average target powder weight of 600 mg/package.

### Example 23 Trimetazidine DiHCl + Betahistine DiHCl Dry Syrup)

Unit Formula

**Table 12**

| | **mg/bottle** | **% (w/w)** | **Function** |
|---|---|---|---|
| **Active Substance** | | | |
| Trimetazidine DiHCl | 140.00 | 1.40 | Drug Substance |
| Betahistine DiHCl | 168.00 | 1.68 | Drug Substance |

| **Excipients** | | | |
|---|---|---|---|
| Xanthan Gum | 85.00 | 0.85 | Viscosity Increasing Agent |
| Citric Acid | 25.00 | 0.25 | Buffering Agent |
| Sodium Citrate | 80.00 | 0.80 | Buffering Agent |
| Sucralose | 205.00 | 2.05 | Sweetener |
| Acesulfame Potassium | 140.00 | 1.40 | Sweetener |
| Colloidal Silicon Dioxide (Aerosil 200) | 155.00 | 1.55 | Anti-Caking Agent / Suspending Agent |
| Microcrystalline Cellulose: Carboxymethylcel lu lose Sodium (Avicel RC-591) | 260.00 | 2.60 | Suspending Agent |
| Mannitol | 8,449.00 | 84.49 | Diluent / Cooling Agent |
| Methylparaben Sodium | 180.00 | 1.80 | Preservative |
| Propylparaben Sodium | 18.00 | 0.18 | Preservative |
| Banana Flavour | 95.00 | 0.95 | Flavouring Agent |
| **Total Weight in Bottle** | **10,000.00** | **100.00** | |

### Example 24 (Manufacturing Process of Example 23)

Manufacturing Process:
1. Trimetazidine DiHCl, Betahistine DiHCl, Citric Acid, Sodium Citrate, Xanthan Gum, Sucralose, Acesulfame Potassium, Aerosil 200, Avicel RC-591, Methylparaben Sodium, Propylparaben Sodium and Banana Flavour are mixed.
2. Powder mixture in Step 1 is sieved through 0.5 mm sieve and mixed again.
3. ¼ of Mannitol, powder mixture in Step 2 and ¼ of Mannitol are mixed.
4. ¼ of Mannitol, powder mixture in Step 3 and ¼ of Mannitol are mixed.
5. Powder mixture in Step 4 is filled into suitable packaging material, such as Type III glass bottles or HDPE bottles, having average target powder weight of 10,000 mg/bottle.

## Claims

1. A pharmaceutical composition or combination comprising; **i)** therapeutically effective amount of betahistine or pharmaceutically acceptable salt thereof; and **ii)** therapeutically effective amount of trimetazidine or pharmaceutically acceptable salt thereof; and **iii)** a pharmaceutically acceptable carrier and/or diluent or mixtures thereof.

2. In pharmaceutical composition or combination, according to preceding claim, betahistine or pharmaceutically acceptable salt thereof is present in an amount of from 4 mg to 300 mg, preferably in an amount of from 6 mg to 30 mg and most preferably 24 mg and trimetazidine or pharmaceutically acceptable salt thereof is present in an amount of from 10 mg to 80 mg, preferably in an amount of from 15 mg to 60 mg and most preferably 20 mg.

3. According to claim 2, a pharmaceutical composition or combination is used in manufacturing a medicament for preventing or treating of: **i)** Ménière's disease and/or, **ii)** Vertigo, tinnitus and hearing loss associated with Ménière's syndrome.

4. According to preceding claims, pharmaceutical composition or combination is prepared by a method; **i)** both active agents are separately granulated and then granulates are combined or, **ii)** one active agent is granulated and then granulate is combined with non granulated active agent or, **iii)** both active pharmaceutical ingredients are granulated together or, **iv)** one active agent is dissolved alone or with one or more excipients and then other active agent alone or with one or more excipients are coated with said solution, **v)** one active agent is dispersed alone or with one or more excipient and then other active agent alone or with one or more excipients are coated or adhered with said dispersion.

5. According to claims of 1 to 2, pharmaceutical composition or combination is prepared by a method : **i)** betahistine and trimetazidine are separately granulated and then they are separately compressed into mini tablets and filled into capsule altogether or, **ii)** betahistine and trimetazidine are separately granulated and then they are separately compressed into tablet and filled into capsule or, **iii)** one of the active agents, betahistine or trimetazidine, is granulated and compressed into tablet or mini-tablets and then combined with granules of other active agent, betahistine or trimetazidine, into capsule or **iv)** betahistine and trimetazidine are separately granulated and then directly filled into capsule without tabletting or, **v)** one of the active agents, betahistine or trimetazidine, is prepared through direct compression into tablet or mini-tablets and then combined with granules of other active agent, betahistine or trimetazidine, into capsule.

6. According to claims of 1 to 2, pharmaceutical composition or combination is in the form of tablet-in-tablet which comprises:
**a)** a core tablet comprising:
trimetazidine or pharmaceutically acceptable salts thereof and a pharmaceutically acceptable carrier or carriers and, **b)** a compressed outer tablet layer comprising: betahistine or pharmaceutically acceptable salts thereof and a pharmaceutically acceptable carrier or carriers **or:a)** a core tablet comprising: betahistine or pharmaceutically acceptable salts thereof and a pharmaceutically acceptable carrier or carriers and, **b)** a compressed outer tablet layer comprising: trimetazidine or pharmaceutically acceptable salts thereof and a pharmaceutically acceptable carrier or carriers.

7. According to claims of 1 to 2, pharmaceutical composition or combination is in the form of bilayer tablet which comprises; **a)** a first layer containing trimetazidine or pharmaceutically acceptable salts thereof and a pharmaceutically acceptable carrier or carriers and, **b)** a second layer containing betahistine or pharmaceutically acceptable salts thereof and a pharmaceutically acceptable carrier or carriers **or: a)** a first layer containing betahistine or pharmaceutically acceptable salts thereof and a pharmaceutically acceptable carrier or carriers and **b)** a second layer containing trimetazidine or pharmaceutically acceptable salts thereof and a pharmaceutically acceptable carrier or carriers.

8. According to claims of 1 to 2, pharmaceutical composition or combination is in the form of inlay tablet which comprises **a)** an inner tablet comprising betahistine or pharmaceutically acceptable salts thereof and a pharmaceutically acceptable carrier or carriers and **b)** an outer tablet comprising trimetazidine or pharmaceutically acceptable salts thereof and a pharmaceutically acceptable carrier or carriers **or:a)** an inner tablet comprising trimetazidine or pharmaceutically acceptable salts thereof and a pharmaceutically acceptable carrier or carriers and **b)** an outer tablet comprising betahistine or pharmaceutically acceptable salts thereof and a pharmaceutically acceptable carrier or carriers wherein inner tablet is encompassed by an outer tablet wherein at least a portion of one surface of the inner tablet is not surrounded by outer tablet.

9. Pharmaceutical composition or combination , according to claims of 1 to 2, is in the form of capsule in capsule which consists of **a)** an external capsule comprising trimetazidine or pharmaceutically acceptable salts thereof and a pharmaceutically acceptable carrier or carriers and **b)** internal capsule (inner capsule) comprising betahistine or pharmaceutically acceptable salts thereof and a pharmaceutically acceptable carrier or carriers **or:a)** an external capsule comprising betahistine or pharmaceutically acceptable salts thereof and a pharmaceutically acceptable carrier or carriers and **b)** internal capsule (inner capsule) comprising trimetazidine or pharmaceutically acceptable salts thereof and a pharmaceutically acceptable carrier or carriers wherein smaller size capsule is encapsulated into a larger capsule.

10. Pharmaceutical composition or combination, according to claims of 1 to 2, comprises spray dried granules of trimetazidine or pharmaceutically acceptable salts thereof and spray dried granules of betahistine or pharmaceutically acceptable salts thereof and preferably an excipient or mixtures of excipients.

11. Pharmaceutical composition or combination, according to claims of 1 to 2, is prepared by dry granulation or direct compression or wet granulation or melt granulation or mixtures thereof.

12. Pharmaceutical composition or combination, according to claims of 1 to 2, is in the form of tablet in capsule which comprises trimetazidine or pharmaceutically acceptable salts thereof and betahistine or pharmaceutically acceptable salts thereof and preferably an excipient or mixtures of excipients.

13. Pharmaceutical composition or combination, according to claims of 1 to 2, is in the form of effervescent tablet or effervescent granule or effervescent pellet or effervescent dry powder which comprises trimetazidine or pharmaceutically acceptable salts thereof and betahistine or pharmaceutically acceptable salts thereof and preferably an excipient or mixtures of excipients.

14. According to claims 1 to 2, pharmaceutical combination is in the form of kit which comprises i) therapeutically effective amount betahistine and optionally an acceptable carrier or diluent in a unit dosage form and ii) therapeutically effective amount of trimetazidine and an acceptable carrier or diluent in a unit dosage form and iii) a container.

15. Pharmaceutical composition or combination, according to claims of 1 to 2, is readily dispersible or readily dissolved granules.
